Europäisches Patentamt

European Patent Office

Office européen des brevets

(19)

(11) Publication number: **0 068 719**
A2

(12) **EUROPEAN PATENT APPLICATION**

(21) Application number: 82303122.4

(22) Date of filing: 16.06.82

(51) Int. Cl.³: **C 12 N 15/00**, C 12 P 21/02, C 07 H 21/04, C 07 C 103/52, C 12 N 1/20, A 61 K 39/29 // C12R1/19

(30) Priority: 16.06.81 JP 93548/81
19.05.82 JP 85280/82

(43) Date of publication of application: 05.01.83
Bulletin 83/1

(84) Designated Contracting States: BE CH DE FR IT LI NL SE

(71) Applicant: Takeda Chemical Industries, Ltd., 27, Doshomachi 2-chome, Higashi-ku Osaka, 541 (JP)

(72) Inventor: Igarashi, Koichi, C3-303, 3 Takenodai Nagaokakyo, Kyoto 617 (JP)
Inventor: Onda, Haruo, 21-6, Aza-junmatsu Tada-in, Kawanishi Hyogo 666-01 (JP)
Inventor: Kikuchi, Masakazu, 4-16, Higashitokiwadai 7-chome Toyono-cho, Toyono-gun Osaka 563-01 (JP)
Inventor: Fujisawa, Yukio, 37-413, 1 Nakamiyakita-machi, Hirakata Osaka 573 (JP)

(74) Representative: Laredo, Jack Joseph et al, Elkington and Fife High Holborn House 52/54 High Holborn, London, WC1V 6SH (GB)

(54) Deoxyribonucleic acids, their production and use.

(57) A recombinant DNA containing a structural gene coding for hepatitis B virus subtype adw surface antigen is constructed. This structural gene is expressed in a transformant containing said recombinant DNA to produce hepatitis B virus subtype adw surface antigen which is useful as a vaccine against hepatitis B.

EP 0 068 719 A2

- 1 -

## Deoxyribonucleic Acids, Their Production and Use

This invention relates to novel deoxyribonucleic acids, their production and use. More particularly, this invention relates to novel deoxyribonucleic acids containing a structural gene coding for hepatitis B virus subtype adw surface antigen, their production and use.

Hepatitis B is a viral disease encountered frequently especially in Tropical Africa, Southeastern Asia and Far East. It is epidemically suggested that hepatitis B may cause chronic hepatitis or liver cirrhosis or further primary liver cancer. The pathogen is a kind of DNA virus, i.e. hepatitis B virus (hereinafter referred to as "HBV"), which occurs as spherical particles having a diameter of 42 nm called Dane particles after the discoverer. In the surface layer therof, there exists HBV surface antigen (hereinafter called "HBsAg"), which is divided into such subtypes as adr, adw, ayr and ayw according to differences in antigenicity [Am. J. Med. Sci., 270, 161-171(1975) which is hereby incorporated by reference]. The subtypes found in Japan are adw and adr.

In the blood of hepatitis B patients, there have been detected not only Dane particles but also smaller particles and tubular particles, and HBsAg of the same type as that found in Dane particles is present also in these particles. Also for other viral species, it is known that an antibody against a viral surface antigen can prevent infection with the virus. In the case of HBV as well, it is expected that a vaccine against hepatitis B might be produced with use of HBsAg. However, HBV infection can occur only in primates

- 2 -

(e.g. humans and chimpanzees) and consequently any attempts to cause infection in cultured cells have failed. Therefore, HBsAg is obtainable only from the blood of virus-infected primates. The smaller particles obtained can meet only the demand of material for diagnostic reagents but cannot afford sufficiently vaccine production.

Recent advances in molecular biology have made it possible to transform bacteria with a DNA coding for a non-bacterial protein. If the expression of the structural gene coding for HBsAg in a bacterium is rendered possible by this gene manipulation technique, large-scale production of HBsAg free from the risk of HBV infection becomes possible and a way to practical use of hepatitis B vaccine is opened.

For the subtype ayw found predominantly in Europe and America among the currently known four subtypes, adw, adr, ayw and ayr, the locus and base sequence of the HBsAg gene have been determined [F. Galibert et al., Nature, 281, 646-650 (1979); and P. Charnay et al., Nucleic Acids Res., 7, 335-346 (1979) which are hereby incorporated by reference] and its expression as a hybrid protein in Escherichia coli has been reported [P. Charnay et al., Nature, 286, 893-895 (1980) which is hereby incorporated by reference].

However, for the adw subtype found predominantly in Japan, neither the DNA base sequence of the HBsAg gene nor the locus thereof on the genome has been reported. Any success in constructing a recombinant DNA containing the gene has not been reported.

In such a background of technological situation, the present inventors have succeeded in constructing a recombinant DNA comprising a structural gene coding for hepatitis B virus subtype adw surface antigen (hereinafter briefly referred to as "HBsAg/adw gene"), have determined the DNA base sequence of said gene and locus thereof on the genome, have obtained a transformant containing said recombinant DNA and have succeeded in expressing HBsAg/adw gene in said transformant.

Thus, the principal object of this invention is to provide the novel DNAs containing HBsAg/adw gene, the transformants containing them, and the industrially feasible method of producing hepatitis B virus subtype adw surface antigen (hereinafter briefly referred to as "HBsAg/adw") by growing said transformants. Other objects of the present invention will be made clear from the description and claims presented hereinafter.

Brief Description of the Drawings

Figure 1 depicts the construction of plasmid pRB322-EcoRI/HBV933 containing HBV/adw DNA.

Figure 2 depicts the construction of plasmid pBR322-Bam HI/HBV971 containing HBV/adw DNA.

Figure 3 depicts plasmid pBR322-EcoRI/HBV933.

Figure 4 depicts the base sequence of HBV BamHI 1355 b DNA fragment and the amino acid sequence of HBsAg/adw.

Figure 5 depicts the restriction enzyme cleavage map of HBV BamHI 1355 b DNA fragment and the fragments obtained from said fragment.

Figure 6 depicts the construction of expression plasmids using trp promoter.

Figure 7 depicts the construction of expression plasmids using the rec A promoter.

The symbols as used herein in the specification, claims and drawings have the meanings shown below in Table 1.

Table 1

| | |
|---|---|
| DNA | = deoxyribonucleic acid |
| A | = adenine |
| T | = thymine |
| G | = guanine |
| c | = cytosine |
| RNA | = ribonucleic acid |
| dATP | = deoxyadenosine triphosphate |
| dTTP | = deoxythymidine triphosphate |
| dGTP | = deoxyguanosine triphosphate |
| dCTP | = deoxycytidine triphosphate |

- 4 -

```
ATP    = adenosine triphosphate
EDTA   = ethylenediamine tetraacetate
SDS    = sodium dodecyl sulfate
Gly    = glycine
Ala    = alanine
Val    = valine
Leu    = leucine
Ile    = isoleucine
Ser    = serine
Thr    = threonine
Cys    = cysteine
Met    = methionine
Glu    = glutamic acid
Asp    = aspartic acid
Lys    = lysine
Arg    = arginine
His    = histidine
Phe    = phenylalanine
Tyr    = tyrosine
Trp    = tryptophan
Pro    = proline
Asn    = asparagine
Gln    = glutamine
b      = base(s)
bp     = base pair(s)
kb     = kilobase(s)
kbp    = kilobase pair(s)
```

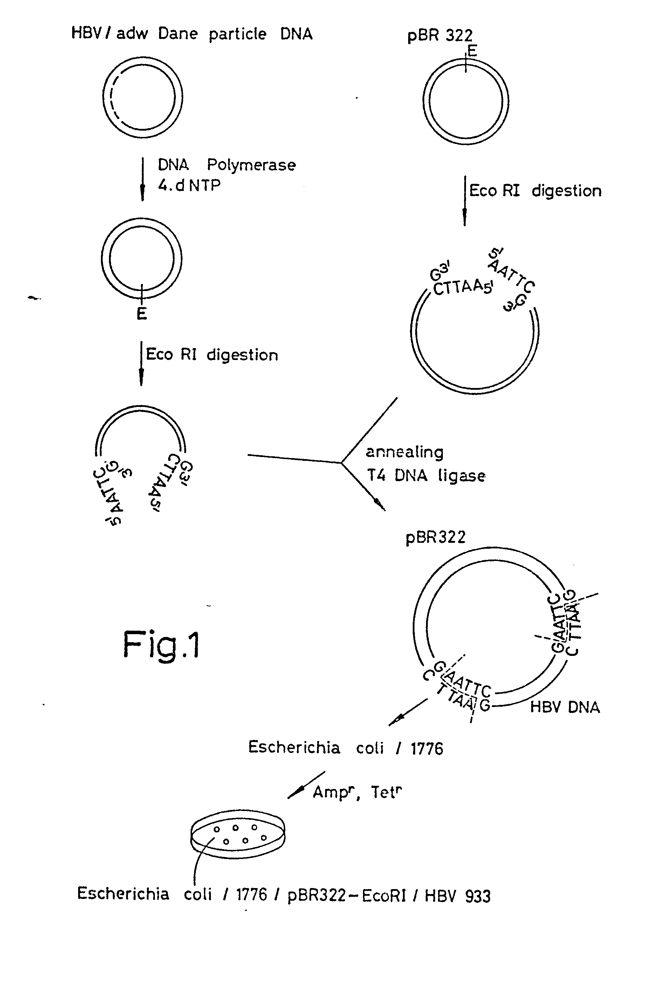

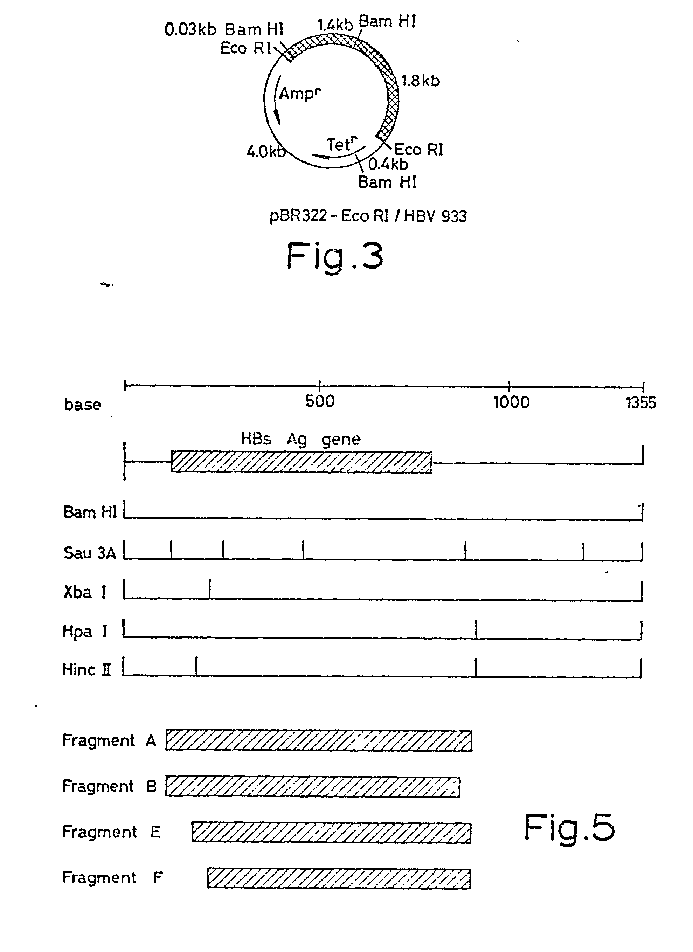

The recombinant DNA of the present invention which contains the HBsAg/adw gene can be produced, for example, by cloning the HBV subtype adw DNA. Thus, for instance, as shown in Figure 1, the HBV/adw Dane particle DNA containing a single-stranded portion is converted to a completely double-stranded DNA by making use of endogenous DNA polymerase reaction, and the latter is then cleaved with restriction enzyme EcoRI. Separately, pBR322 DNA, which is a plasmid, is cleaved with the same enzyme EcoRI. Both the cleavage products are ligated together at the respective EcoRI sites with T4 DNA ligase. From among colonies of Escherichia coli χ1776 strain transformed with the ligation product, there can be selected an ampicillin- and tetracycline-resistant transformant (χ1776/pBR322-EcoRI/HBV933) containing plasmid pBR322-EcoRI/HBV933 with a 3.2 kbp HBV/adw DNA inserted therein (Figures 1 and 3).

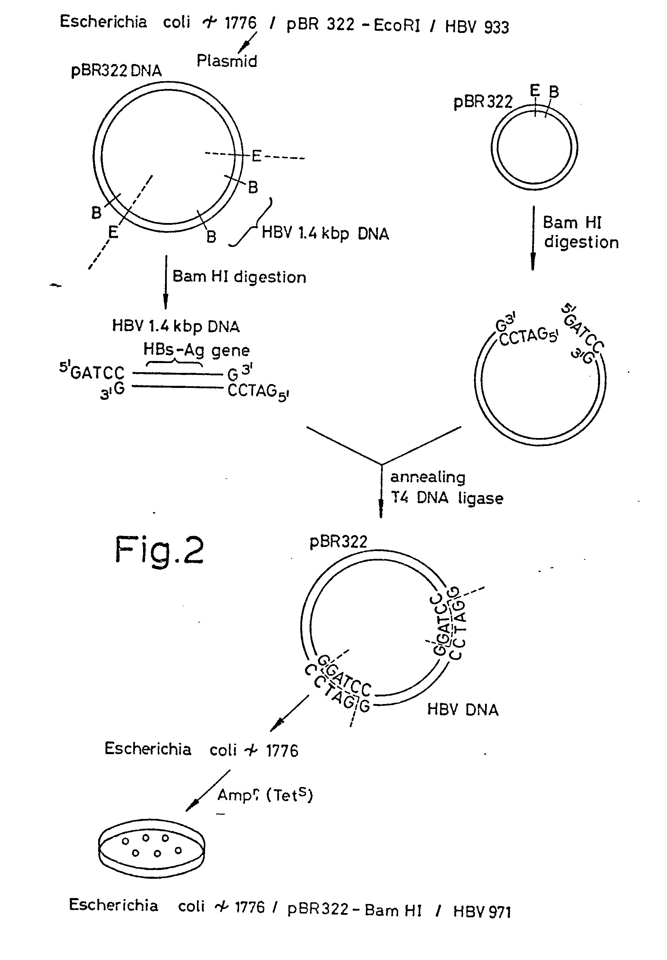

The above plasmid pBR322-EcoRI/HBV933 can be prepared in large quantities by growing the above-mentioned transformant in an appropriate medium such as L broth. When the plasmid is digested with restriction enzyme BamHI, three DNA fragments are obtained as shown in Figure 2. Among the resulting DNA fragments, the one which carries the HBsAg/adw gene and contains about 1.4 kbp (determined as being 1355p by the DNA sequencing analysis) is isolated and inserted into pBR322 at the BamHI site, to give a plasmid (pBR322-BamHI/HBV971). The χ1776 strain of Escherichia coli is transformed with this plasmid. By selecting and growing the thus-transformed χ1776/pBR322 -BamHI/HBV971, a large amount of said plasmid can be obtained.

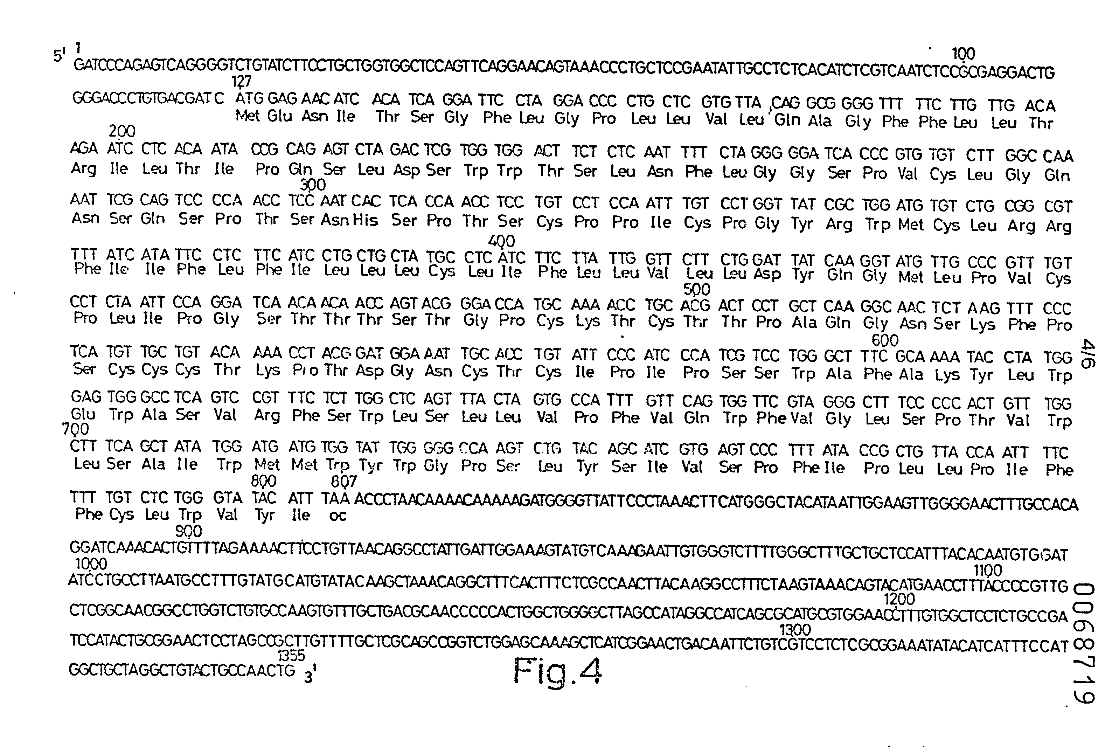

The HBsAg gene exists on the 1355 b DNA fragment, which has the base sequence shown in Figure 4. Translation of the 678 bases following the start codon ATG, which begins with base 127, and preceding the stop codon TAA beginning with base 805 gives the corresponding amino acid (Figure 4) which has N-terminal and C-terminal amino acid sequences identical with those reported for HBsAg/adw by Peterson et al. [Proc. Natl. Acad. Sci. USA, 74, 1530-1534 (1977) which is hereby incorporated by reference]. It is therefore concluded that the gene coding for HBsAg/adw covers bases 127-807 of the base sequence shown in Figure 4.

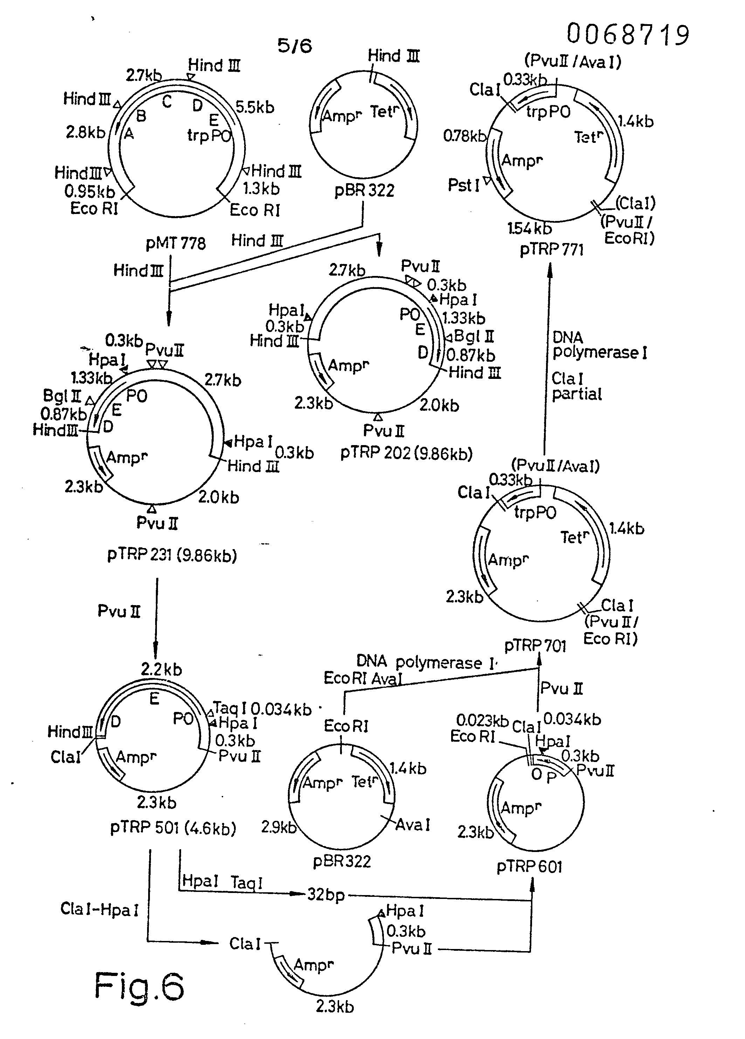

From the above 1355 b BamHI DNA fragment, a gene fragment containing the whole or part of the HBsAg gene base sequence can be excised by using appropriate restriction enzymes. For instance, as shown in Figure 5, digestion of said BamHI DNA fragment with restriction enzyme HpaI gives a 918 b DNA fragment, which is then partially cleaved with restriction enzyme Sau3A to give a 792 bp DNA fragment (hereinafter called "fragment A" for short) covering bases 123-918 and a 761 bp DNA fragment (hereinafter called "fragment B" for short) covering bases 123-887 in the base sequence. Similarly, a 727 bp DNA fragment (hereinafter called "fragment E" for short) covering bases 192-918 and a 695 bp DNA fragment (hereinafter called "fragment F" for short) covering bases 220-918 can be excised respectively by digestion of the BamHI DNA fragment with restriction enzyme HincII and by double digestion with restriction enzymes HincII and XbaI.

The HBsAg/adw gene for which the DNA base sequence and the locus on the HBV genome have been determined as described above is different in DNA base sequence from the so-far reported HBsAg genes. Furthermore, as is evident from the DNA base sequence shown in Figure 4, the thus-obtained HBsAg/adw gene contains no intron therewithin. This means that the gene can be transcribed as it is to lead to phenotypic expression as a messenger RNA in animal cells as well as in bacterial cells. Therefore, introduction of this gene into an animal cell, for example, a human liver cell, by the calcium-phosphate method [Virology, 52, 456-467 (1973) which is hereby incorporated by reference], for instance, may lead to HBsAg/adw synthesis within said cell. Furthermore, this HBsAg/adw gene with an appropriate expression system is inserted into a plasmid DNA, introduced, for example, into cells of Escherichia coli, and cultivation of the bacterial cells may enable low-cost mass production of HBV infectivity-free HBsAg/adw.

Advantageously, a recombinant DNA capable of expressing the HBsAg/adw gene is constructed by inserting the whole necleotide sequence of the HBsAg/adw gene or a fragment thereof into the gene coding for anthranilic acid synthetase component I or anthranilic acid synthetase component II in the tryptophan operon, the gene coding for rec A protein in the rec A operon or the gene coding for β-lactamase in the

- 7 -

ampicillin resistance gene operon, or downstream from the tryptophan promoter or rec A promoter. Among such recombinant DNAs, the one in which a vector with the trp promoter inserted therein is used, can be constructed,for example, in the following manner (cf Figure 6). Plasmid pMT778 DNA containing the tryptophan operon inserted therein is digested with restriction enzyme HindIII, whereby a 5.5 kbp DNA fragment containing trp promoter, operator, trp E and part of trp D can be excised. The fragment is inserted into plasmid pBR322 DNA at the HindIII site thereof to give plasmid pTRP 202 and pTRP231 DNAs. In each of these plasmids, there is a restriction enzyme BglII cleavage site on the codons (GAG ATC TAC) for amino acids 322-324 as encoded in trp E, and therefore insertion of the whole HBsAg gene or a fragment thereof into the plasmid at said site leads to formation of a fused gene with the gene (trp E) coding for anthranilic acid synthetase component I in the tryptophan operon. Digestion of plasmid pTRP231 DNA with restriction enzyme PvuII followed by religation with T4 DNA ligase gives a 4.8 kbp plasmid pTRP501 DNA. In this plasmid, there is a restriction enzyme HindIII cleavage site in the connection (GAAGCTTA) between trp D and plasmid pBR322 and consequently insertion of the whole HBsAg gene or a fragment thereof at this site leads to formation of a fused gene with the gene (trp D) coding for anthranilic acid synthetase component II in the tryptophan operon. Double digestion of plasmid pTRP501 DNA with restriction enzymes ClaI and HpaI gives a 2.6 kbp ClaI-HpaI DNA fragment, and double digestion of said plasmid DNA with restriction enzymes HpaI and TaqI gives a 32 bp DNA fragment. Ligation of the 32 bp DNA fragment with the above 2.6 kbp DNA by means of T4 DNA ligase leads to formation of plasmid pTRP601 DNA. A 1.4 kbp DNA fragment obtainable from plasmid pBR322 DNA by double digestion with restriction enzymes EcoRI and AvaI is filled in with DNA polymerase I large fragment (also called Klenow's DNA polymerase I) and inserted into the above plasmid pTRP601 DNA at the restriction enzyme

- 8 -

PvuII site, whereby a 4.0 kbp plasmid pTRP701 is formed. This plasmid DNA is partially digested with restriction enzyme ClaI, and the resulting cohesive ends are filled in with Klenow's DNA polymerase I and then ligated with T4 DNA ligase, whereby a plasmid pTRP771 DNA having only one ClaI cleavage site is formed. In DNAs of the plasmids pTRP601 and pTRP771, the ClaI cleavage site is located downstream from the trp promoter and SD (Shine and Dalgarno) sequence and therefore is available for insertion of the whole HBsAg gene or a fragment thereof with ATG joined to the 5'-end without shifting any reading frame. DNAs of the plasmids pTRP701 and pTRP771 each has a restriction enzyme PstI cleavage site in the β-lactamase gene (bla) in the ampicillin resistance gene operon and the site is also available for insertion of the whole HBsAg gene or a fragment thereof.

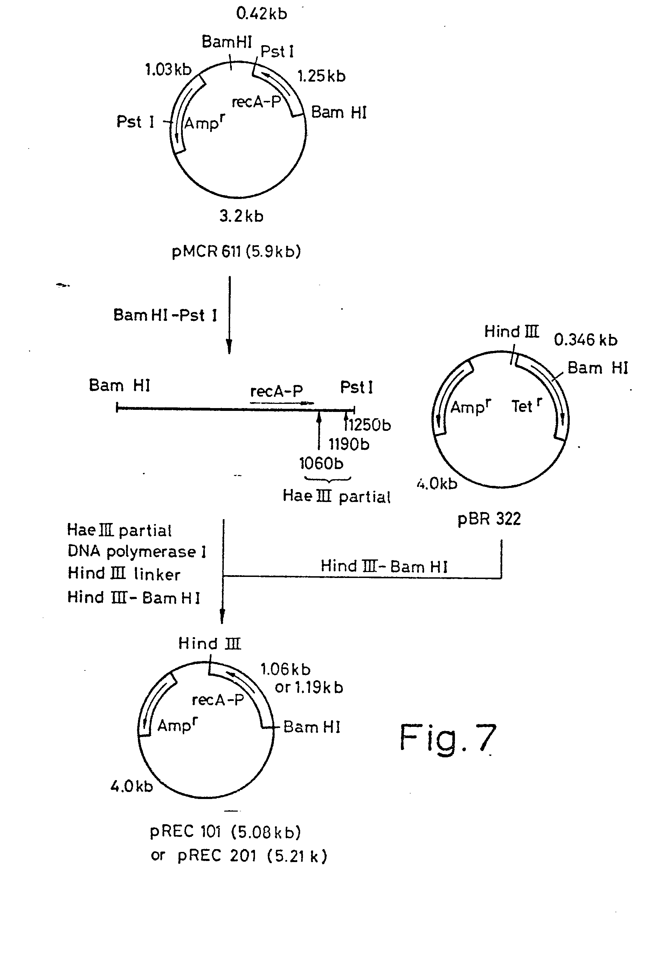

A vector for expression using the rec A promoter can be produced in the following manner (cf Figure 7). Plasmid pMCR611 DNA with the rec A promoter inserted therein [T. Miki et al., Mol. Gen. Genet., 183, 25-31 (1981) which is hereby incorporated by reference] is subjected to double digestion with restriction enzymes BamHI and PstI, whereby a 1.25 kbp DNA fragment containing the rec A promoter can be obtained. Partial cleavage of said fragment with restriction enzyme HaeIII gives a 1.06 kbp DNA fragment containing the rec A promoter and coding for the 15 N-terminal amino acids of rec A protein and a 1.19 kbp DNA fragment containing the rec A promoter and coding for the 59 N-terminal amino acids. These fragments are respectively filled in with Klenow's DNA polymerase I and then ligated with HindIII linker d(CAAGCTTG) at both ends of each fragment under the action of T4 DNA ligase. The fragments each carrying the linkers are cleaved with restriction enzymes BamHI and HindIII and respectively ligated with a 4.0 kbp DNA fragment obtained by double digestion of plasmid pBR322 DNA with restriction enzymes BamHI and HindIII, in the presence of T4 DNA ligase, whereby plasmid pREC101 and pREC201 DNAs .are formed. Since these plasmid DNAs have a restriction enzyme HindIII cleavage site, insertion of the whole HBsAg gene or a fragment thereof into the plasmids at said site leads to formation of fused genes with the gene coding for rec A protein in the rec A operon.

After the insertion of the whole HBsAg gene or a fragment thereof into the expression vectors prepared in the above manner with T4 DNA ligase, the resulting plasmid DNAs each can be introduced into an appropriate host. The host includes, among others, such microorganisms as Escherichia coli, Bacillus subtilis and yeasts and preferably is an Escherichia coli strain (e.g. 294, W3110 or RR1), especially the strain 294.

The transformation of a host with the thus-obtained recombinant DNA can' be conducted by the known methods [S. N. Cohen et al., Proc. Natl. Acad. Sci. USA, 69, 2110-2114 (1972) which is hereby incorporated by reference] or a modification thereof.

For instance, it is possible to transform Escherichia coli 294 by introducing thereinto the HBsAg gene-containing novel recombinant plasmid DNA obtained in the above manner and select a transformant through ampicillin and/or tetracycline resistance as the phenotype. Escherichia coli 294 is a known strain of microorganism [K. Backman et al., Proc. Natl. Acad. Sci. USA, 73, 4174-4178 (1976) which is hereby incorporated by reference] and available also from Institute for Fermentation, Osaka under the deposit No. IFO-14171 (deposited March 23, 1982). For picking out a strain carrying the HBsAg gene-containing novel recombinant plasmid DNA, the following technique, for instance, may be used. The 792 bp fragment A containing the whole HBsAg gene base sequence is labelled with a radioisotope such as $^{32}$P by the nick translation method [P. W. J. Rigby et al., J. Mol. Biol., 113, 237-251 (1977) which is hereby incorporated by reference]. Thereafter, using the labelled product as a probe, a positive clone can be picked out from among the already-obtained drug-resistant transformants by the per se known colony hybridization method [M. Grunstein and D. S. Hogness, Proc. Natl. Acad. Sci. USA, 72, 3961-3965 (1975) which is hereby incorporated by reference].

A transformant picked out in this manner is grown in a per se known medium. The midium may be, for instance, L broth, Penassay broth, or M-9 medium containing glucose and Casamino Acids [J. Miller, Experiments in Molecular Genetics, 431-433 (Cold Spring Harbor Laboratory, New York, 1972) which is hereby incorporated by reference]. If necessary for efficient operation of the promoter, such an agent as 3β-indolylacrylic acid, nalidixic acid or mitomycin C may be added to the medium.

The cultivation is generally carried out at 15-43°C, preferably at 28-40°C, for 2-24 hours, preferably for 4-16 hours, if necessary with aeration and/or agitation.

After the cultivation, the bacterial cells are collected in a known manner, the cells are suspended in a buffer and lysed by lysozyme or ultrasonic treatment, for instance. From the supernatant obtained by centrifugation, the HBsAg may be isolated by a method generally known for protein purification.

The HBsAg activity of the product can be determined by a per se known immunological method, for example, using Auslia II-125 kit (Abbott Laboratories, U.S.A.) [C.M. Ling et al., J. Immuno., 109, 834-841(1972) which is hereby incorporated by reference] or Auszyme II kit (Abbott Laboratories, U.S.A.) [R. Wei et al., Clin. Chem., 23, 813-815(1977) which is hereby incorporated by reference].

The HBsAg/adw or a polypeptide equivalent thereto in immunological or biological activity as produced in accordance with the present invention has immunological and biological activity equivalent to those of the HBsAg/adw produced by the conventional method and can be used for the same purpose for which the conventional HBsAg/adw is used, for example, as a vaccine against hepatitis B or a diagnostic agent, in the same mode of use as known for the conventional product.

The following Examples and Reference Example further illustrate the present invention in more detail. However, they are by no means limitative of the present invention.

Example 1

(1)    Preparation of HBV/adw DNA

From a chimpanzee (female, weighing 12 kg), 73 days after inoculation with HBV/adw [received from Phoenix Laboratories Division, Center for Disease Control (Phoenix, Ariz., USA); Intervirology 3, 378-381 (1974) which is hereby incorporated by reference] when the blood in the chimpanzee was positive for HBsAg and the presence of Dane particles in the plasma was confirmed by electron microscopy, 100 ml of plasma was taken [Intervirology 10, 254-264 (1978) which is hereby incorporated by reference]. This was centrifuged (24,000 rpm, 16 hours, 4°C) using a Spinco SW27 rotor (Beckman, USA) to give HBV/adw as a precipitate. The precipitate was suspended in 30 ml of 20 mM Tris-HCl (pH 7.6) and the suspension was centrifuged again under the same conditions (SW27 rotor, 24,000 rpm, 16 hours, 4°C) so as to wash the HBV, whereby the serum components were removed and the HBV was concentrated. The precipitate was suspended in 5 ml of 20 mM Tris-HCl (pH 7.6) and the suspension stored at -20°C until its use in DNA synthesis reaction.

Using 0.8 ml of the above-mentioned suspension of precipitate and following the procedure of Kaplan et al. [J. Virol., 12, 995-1005 (1973) which is hereby incorporated by reference], the single-stranded portion of the HBV DNA was made double-stranded by incubation in a reaction mixture containing $^3$H-dTTP [160 mM Tris-HCl (pH 7.5), 40 mM MgCl$_2$, 120 mM NH$_4$Cl, 0.3% 2-mercaptoethanol, 0.5% Nonidet P-40, 1.0 mM dATP, dCTP, dGTP] at 37°C for 16 hours. Occurrence of the DNA synthesis reaction was confirmed by using as an index the uptake of $^3$H-dTTP into the acid insoluble fraction. The thus-obtained reaction mixture was centrifuged using a Spinco SW55 rotor (35,000 rpm, 6 hours, 4°C). The HBV

- 12 -

after the DNA synthesis reaction was collected as a precipitate and suspended in 1 ml of buffer for DNA extraction [0.02 M Tris-HCl (pH 7.6), 0.02 M EDTA, 1% SDS, 1 mg/ml Proteinase K] and incubated at 37°C for 16 hours. Deproteinization was performed twice with 1 ml of phenol saturated with 0.01 M Tris-HCl (pH 7.6), 0.1 M NaCl and 0.001 M EDTA [J. Virol. $\underline{23}$, 368-376 (1977) which is hereby incorporated by reference], the aqueous layer was taken, 0.2 M NaCl and 2.5 volumes of 99% ethanol were added thereto, and HBV DNA precipitation was allowed to take place at -20°C. The above procedure gave a double-stranded HBV/adw DNA with the single-stranded portion completely repaired.

(2)    Cloning of HBV/adw DNA

The double-stranded HBV/adw DNA obtained by the procedure mentioned above under (1) was digested with EcoRI, a restriction enzyme capable of recognizing a specific site in the DNA chain and cleaving the DNA to form cohesive termini, at 37°C in the presence of 100 mM Tris-HCl (pH 7.5), 7 mM $MgCl_2$, 50 mM NaCl and 7 mM 2-mercaptoethanol, to give a HBV/adw DNA EcoRI fragment. A plasmid of Escherichia coli, pBR322 [Gene, $\underline{2}$, 95-113 (1977) which is hereby incorporated by reference], was used as the cloning vector. This plasmid is a circular DNA with two drug resistance genes, one for tetracycline resistance and the other for ampicillin resistance, existing thereon. This plasmid DNA is cleaved with EcoRI at one site. Even when another DNA is inserted in said DNA at the EcoRI site, the genes for ampicillin resistance and tetracycline resistance are retained. Therefore, after conversion of the pBR322 DNA to a linear DNA by EcoRI digestion, the 5'-terminal phosphate residue was removed by alkaline phosphatase treatment [Science, $\underline{196}$, 1313-1319 (1977) which is hereby incorporated by reference]. The thus-obtained HBV/adw DNA EcoRI fragment and plasmid DNA each has on either end a single-stranded cohesive terminus resulting from EcoRI digestion. These HBV/adw DNA EcoRI fragment and pBR322 EcoRI fragment were mixed and

- 13 -

subjected to DNA ligation at 14°C under the action of T4 DNA ligase in the presence of 66 mM Tris-HCl (pH 7.6), 6.6 mM $MgCl_2$, 10 mM dithiothreitol and 0.4 mM ATP. In the reaction product obtained in this manner, there is expected the presence of a circular DNA formed by ligation of the linear DNA derived from pBR322 and the HBV/adw DNA through the complementary base sequences on both termini of each DNA. In this case, the preliminary elimination of the 5'-terminal phosphate residue excludes the possibility of recircularization of the linearized pBR322 DNA with T4 ligase. Therefore, Escherichia coli χ1776 strain [ATCC 31244; U.S. Patent No. 4,190,495 which is hereby incorporated by reference] was incubated in a solution (pH 5.6) containing 70 mM $MnCl_2$, 30 mM $CaCl_2$ and 40 mM sodium acetate at 0°C for 20 minutes [J. Mol. Biol., 96, 495-509 (1975) which is hereby incorporated by reference] and then suspended in 100 µl of the same solution (about $5 \times 10^8$ bacterial cells) and thereto was added 5 µl of the reaction mixture obtained by the above-mentioned procedure. After incubation at 0°C for 60 minutes, the bacterial suspension was plated onto an L-broth agar medium (containing,per liter thereof, 10 g Bacto tryptone, 5 g Bacto yeast extract, 10 g NaCl and 0.8 g glucose) containing 20 µg/ml ampicillin, 50 µg/ml thymidine and 20 µg/ml diaminopimelic acid and incubated at 37°C for 2 days. Among the ampicillin resistance colonies which formed, those which contained the pBR322 plasmid carrying the exogenous DNA inserted therein were selected by the "toothpick" assay [Science, 195, 393-394 (1977) which is hereby incorporated by reference]. Thus, those colonies in which bacterial cells contained a larger plasmid than pBR322 were selected and collected by picking up such colonies with a toothpick and suspending the cells in 100 µl of a solubilizing solution (1% SDS, 10% glycerol, 0.02 M EDTA, 0.04% xylene cyanol), followed by heat-treating at 70°C for 10 minutes and subjecting to 1% agarose gel electrophoresis. In this way, the cloning of HBV/adw DNA was completed.

- 14 -

(3)    Properties of cloned HBV/adw DNA

For measuring the size of the HBV/adw DNA cloned in the above manner, the Escherichia coli cells containing the recombinant DNA as collected in paragraph (2) were grown in an L-broth medium containing 20 µg/ml ampicillin, 50 µg/ml thymidine and 20 µg/ml diaminopimelic acid, with the addition of chloramphenicol to the concentration of 170 µg/ml in the logarithmic growth phase. The cultivation was continued for several hours for amplifying the plasmid DNA. The cells were then lysed with lysozyme, EDTA and SDS, and the plasmid DNA was separated and purified by cesium chloride density gradient centrifugation [J. Bact., 121, 354-362 (1975) which is hereby incorporated by reference]. Digestion of the thus-obtained plasmid DNA with EcoRI gave a DNA with 3.2 kbp and a DNA with 4.3 kbp alone. The 4.3 kbp DNA is the vector DNA, namely the pBR322 DNA. Therefore, the 3.2 kbp DNA is the DNA cloned as the HBV/adw DNA. When digested with BamHI, a restriction enzyme, the 3.2 kbp HBV/adw DNA was cleaved to give a DNA with about 1.8 kbp, a DNA with 1.4 kbp and a DNA with 30 bp. The χ1776 strain containing the recombinant DNA from the 3.2 kbp HBV DNA and the pBR322 DNA as obtained in the manner mentioned above was named χ1776/pBR322-EcoRI/HBV933. Furthermore, the 1.4 kbp HBV DNA was taken from the recombinant pBR322-EcoRI/HBV933 by the above technique and inserted in the pBR322 DNA at the BamHI site by the same procedure as mentioned above under paragraph (2), for cloning the 1.4 kbp DNA. Since the pBR322 DNA has one BamHI cleavage site which is on the tetracycline resistance gene, drug-sensitive bacterial cells transformed with a plasmid derived from the pBR322 by insertion of some other DNA at the BamHI site exhibits ampicillin resistance and tetracycline sensitivity, so that the strain can easily be distinguished from cells containing pBR322 without said other DNA inserted therein (ampicillin-resistant and tetracycline-sensitive cells). Therefore, from among the colonies which exhibited ampicillin resistance after transformation by the procedure mentioned above, tetracycline-sensitive colonies were selected and then those Escherichia coli cells carrying the pBR322 plasmid with the exogenous DNA (HBV DNA fragment) inserted therein were chosen and collected by the "toothpick" assay [Science, 195, 393-394 (1977) which is hereby incorporated by reference] (cf Figure 2). The thus-obtained Escherichia coli χ1776 strain containing the plasmid pBR322 with the 1.4 kbp HBV DNA inserted therein was named χ1776/pBR322-BamHI/HBV971.

(4)    Identification of HBsAg/adw gene

For confirming the locus of the HBsAg/adw gene supposedly existing on the HBV/adw DNA cloned as described above under paragraphs (2) and (3), the base sequence of a part of this DNA was determined. First, the χ1776/pBR322-EcoRI/HBV933 strain was grown by the procedure mentioned above under paragraph (3), the plasmid DNA being amplified by using chloramphenicol. The pBR322-EcoRI/HB933 was isolated and purified, and digested with EcoRI and BamHI. The 1.4 kbp HBV DNA was separated by 1.0% agarose gel electrophoresis and eluted from the agarose gel. The DNA sequencing of this 1.4 kbp fragment was carried out by using the M13 chain terminating sequencing system [Bethesda Research Laboratories, USA; Proc. Natl. Acad. Sci. USA, 74, 5463-5467 (1977); Nucleic Acids Res., 8, 1731-1743 (1980); and M13 cloning/"dideoxy" sequencing manual published by BRL Inc., Maryland,U.S.A. which are hereby incorporated by reference] in a manner summarized below. First, the 1.4 kbp DNA was cloned through insertion into the phage M13mp7RF DNA at the BamHI site. The DNA was allowed to mature in the form of a phage particle through infection of Escherichia coli K12 JM103 strain therewith. A single-stranded phage DNA carrying one DNA strand of the 1.4 kbp HBV DNA as inserted therein was extracted from the phage and purified. Using this as the template, a very short single-stranded DNA, which had a base sequence complementary to that of the portion adjacent to the inserted 1.4 kbp DNA, as the primer

- 16 -

and dATP, dTTP, dGTP and dCTP as the substrates (one to four of the substrates being $^{32}$P-labelled), the DNA synthesis reaction was performed. In case four DNA synthesis inhibitors, ddATP, ddTTP, ddGTP and ddCTP, at adequate concentrations are respectively added in conducting such synthesis, the DNA synthesis is terminated at the time when the relevant inhibitor is taken up into the DNA. Therefore, the DNA chain was separated into single-stranded chains, which were analyzed by 8% acrylamide gel electrophoresis and radioautography. In this manner, the 3'-terminal base identification depending on the length of the DNA synthesized could successfully be carried out. In the same manner, DNA sequencing was conducted also for the DNA fragment obtained by digesting the 1.4 kbp HBV DNA with Sau3A, a restriction enzyme. In this manner, the whole base sequence of the 1.4 kbp HBV DNA was determined as shown in Figure 4. The base sequence shown in Figure 4 is numbered starting from the 5'-terminal base of the 1.4 kbp HBV DNA, and the amino acid sequence of the polypeptide to be translated from the HBsAg/ adw gene is shown under the DNA base sequence. Thus, it is shown that the HBsAg/adw gene has the base sequence covering bases 127-807 and that the molecular weight of the polypeptide coded for by this gene is about 25,000.

## Example 2

Preparation of plasmid pBR322-EcoRI/HBV933

A loopful of a transformant χ1776/pBR322-EcoRI/HBV933 carrying plasmid pBR322-EcoRI/HBV933 with HBV/adw DNA inserted therein as obtained in above Example 1-(3) was inoculated into 30 ml of L broth containing 100 µg/ml diaminopimelic acid, 20 µg/ml thymidine and 12.5 µg/ml tetracycline and cultivated overnight at 37°C with shaking at 200 rpm. A 25-ml portion of the culture broth was transferred to 500 ml of L broth placed in a one-liter flask and containing 100 µg/ml diaminopimelic acid and 20 µg/ml thymidine and cultivated at 37°C for 4 hours with shaking at 200 rpm. Then, chloramphenicol was added in a final

concentration of 170 µg/ml and the cultivation was continued for further 6-8 hours so as to amplify the plasmid DNA. A total of 3 liters of the culture broth cultivated under the same conditions was centrifuged at 8,000 rpm at 4°C for 5 minutes, and the cells obtained were washed twice with saline and suspended in 30 ml of a buffer [50 mM Tris-HCl (pH 8.0), 25% (w/v) sucrose]. Thereto was added 6 ml of a 5 mg/ml lysozyme solution, the mixture was placed on ice, and 12 ml of 0.2 M $Na_2EDTA$ (pH 8.0) was added thereto. Then, following further addition of 15 ml of 5 M NaCl and 6 ml of 10% SDS, the mixture was kept at 4°C for more than an hour and then centrifuged at 18,000 rpm at 4°C for 30 minutes. To the thus-obtained supernatant, there were added 100-200 µg/ml of ethidium bromide (EtBr) and a sufficient amount of cesium chloride (CsCl) to make the density 1.57-1.58 g/cm$^3$. The resulting mixture was subjected to CsCl-EtBr equilibrium density gradient centrifugation at 40,000 rpm at 20°C for 48 hours using a Beckman 50 Ti rotor. The plasmid DNA band fraction was collected and, for purification, further subjected to CsCl-EtBr equilibrium density gradient centrifugation under the same conditions. After the centrifugation, the plasmid DNA fraction was collected and, following removal of the EtBr by addition of an equal volume of iso-amyl alcohol, dialyzed in a buffer (10 mM Tris-HCl, pH 8.0, 1 mM EDTA). The dialyzed solution was used as plasmid pBR322-EcoRI/HBV933 DNA sample.

### Example 3

#### Preparation of HBsAg/adw DNA fragments

The HBsAg/adw gene was prepared from the plasmid pBR322-EcoRI/HBV933 DNA in the following manner. 400 µg of plasmid pBR322-EcoRI/HBV933 DNA was cleaved with restriction enzyme BamHI (Takara Shuzo Co., Ltd., Japan) at 30°C for 6 hours in 800 µl of a reaction mixture (10 mM Tris-HCl, pH 8.0, 7 mM $MgCl_2$, 100 mM NaCl, 2 mM 2-mercaptoethanol, 0.01% bovine serum albumin, 100 units BamHI). The reaction mixture was subjected to electrophoresis at 160 V for 2 hours

- 18 -

using 1.0% agarose (Seakem) slab gel in a buffer [100 mM Tris-HCl, 100 mM boric acid, 2 mM EDTA (pH 8.3)]. Thereafter, a gel fragment containing a 1.4 kbp DNA fragment was sealed in a Visking tube, which was immersed in the buffer for electrophoresis. In this manner, the DNA fragment was eluted from the gel electrically [M. W. McDonell et al., J. Mol. Biol., 110, 119-146 (1977) which is hereby incorporated by reference]. The dialyzed solution was taken out and extracted with phenol and further with ether, then NaCl was added to a concentration of 0.2 M, thereafter 2 volumes of cold ethanol was added, and the DNA was allowed to precipitate at -20°C.

50 µg of the 1.4 kbp DNA fragment was cleaved with restriction enzyme HpaI (Takara Shuzo) at 37°C overnight in a reaction mixture (10 mM Tris-HCl, pH 7.5, 7 mM $MgCl_2$, 100 mM KCl, 7 mM 2-mercaptoethanol, 0.01% bovine serum albumin, 5 units HpaI). Then, following addition of a diluted solution of restriction enzyme Sau3A, partial digestion was performed at 37°C for 15 minutes. The reaction mixture was electrophoresed using 1.2% agarose slab gel under the same conditions as mentioned above. After the electrophoresis, the 792 bp fragment A and 761 bp fragment B each containing the whole base sequence of the HBsAg gene were respectively eluted under the same conditions as mentioned above. Each eluate was extracted with phenol and ether, NaCl was added to a concentration of 0.2 M and then 2 volumes of cold ethanol was added for the precipitation of each DNA.

5 µg of the 1.4 kbp DNA fragment was cleaved with restriction enzyme HincII (Takara Shuzo) at 37°C for 2 hours in 30 µl of a reaction mixture (10 mM Tris-HCl, pH 8.0, 7 mM $MgCl_2$, 60 mM NaCl, 7 mM 2-mercaptoethanol, 5 units HincII) and then electrophoresed using 1.0% agarose slab gel under the same conditions as described above. After the electrophoresis, the 727 bp fragment E containing a fragment of the HBsAg gene was eluted from the gel under the same conditions as mentioned above. To the eluate after extraction with phenol and ether, NaCl was added to a concentration of 0.2 M and then 2 volumes of cold ethanol was added so as to cause precipitation of the DNA.

5 µg of the 1.4 kbp DNA fragment was cleaved with restriction enzymes XbaI (New England BioLabs, Great Britain) and HpaI in 30 µl of a reaction mixture (6 mM Tris-HCl, pH 7.9, 6 mM MgCl$_2$, 150 mM NaCl, 0.01% bovine serum albumin, 5 units XbaI, 5 units HpaI) at 37°C for 2 hours and then electrophoresed using 1.0% agarose slab gel under the same conditions as mentioned above. After the electrophoresis, the 695 bp fragment F containing a fragment of the HBsAg gene was eluted from the gel under the same conditions as mentioned above. To the eluate after treatment with phenol and ether, NaCl was added to a concentration of 0.2 M and then cold ethanol was added so as to cause precipitation of the DNA.

Reference Example    Construction of vector for expression
                     of HBsAg/adw gene

(i)    Construction of trp promoter-containing expression
       vector

(1)    2 µg of plasmid pMT778 DNA with the tryptophan operon inserted therein was cleaved with restriction enzyme HindIII (Takara Shuzo) at 37°C for 2 hours in 20 µl of a reaction mixture (10 mM Tris-HCl, pH 7.5, 7 mM MgCl$_2$, 60 mM NaCl, 2 units HindIII) and then electrophoresed using 0.7% agarose slab gel under the same conditions as described in Example 3. After the electrophoresis, a 5.5 kbp DNA fragment containing trp promoter, operator, trp E and part of trp D was recovered from the gel by the procedure of Exmaple 3.

1 µg of plasmid pBR322 DNA was converted to a linear DNA form by digestion with restriction enzyme HindIII in 20 µl of a reaction mixture (10 mM Tris-HCl, pH 7.5, 7 mM MgCl$_2$, 60 mM NaCl, 2 units HindIII) at 37°C for 2 hours, and then treated with 0.1 unit of alkaline phosphatase

0068719

- 20 -

(Worthington, U.S.A.) at 65°C for 30 minutes, whereby the 5'-terminal phosphate residue was removed. The reaction mixture was deproteinized with phenol, and the DNA (HindIII-digested pBR322) was precipitated with cold ethanol.

The HindIII-digested pBR322 DNA (400 ng) and the above 5.5 kbp DNA fragment (200 ng) were mixed and treated for DNA ligation in 10 µl of a reaction mixture [66 mM Tris-HCl, pH 7.6, 6.6 mM $MgCl_2$, 10 mM dithiothreitol, 1 mM ATP, 2 units T4 DNA ligase (Takara Shuzo)] at 14°C overnight. The reaction mixture was used for transformation of Escherichia coli 294 by the above-cited method of Cohen et al. Among the transformants selected on the basis of ampicillin resistance as an index, there were found a strain resistant only to ampicillin and a strain resistnat to both ampicillin and tetracycline. Plasmid DNA from the cells of each strain by the method of Birnboim and Doly [H. C. Birnboim and J. Doly, Nucleic Acids Res., 7, 1513-1523 (1973) which is hereby incorporated by reference] was isolated and the molecular weight and restriction enzyme cleavage pattern were investigated in detail. Plasmid pTRP231 DNA with the 5.5 kbp DNA fragment inserted therein in the same direction as the β-lactamase gene and plasmid pTRP202 with said DNA fragment inserted therein in the same direction as the tetracycline resistance gene were detected in the former strain and in the latter one, respectively (cf Figure 6).

(2) 1 µg of plasmid pTRP231 DNA was cleaved with restriction enzyme PvuII (Takara Shuzo) in 20 µl of a reaction mixture (10 mM Tris-HCl, pH 7.5, 7 mM $MgCl_2$, 60 mM NaCl, 7 mM 2-mercaptoethanol, 2 units PvuII) at 37°C for 2 hours. The reaction mixture was deproteinized with phenol, and DNA was precipitated with cold ethanol. This DNA was religated with T4 DNA ligase under the conditions described in above (i)(1). The reaction mixture was used for transformation of Escherichia coli 294. Among ampicillin-resistant transformants, there was obtained plasmid pTRP501 in which a ca. 5.1 kbp PvuII DNA fragment of plasmid pTRP231 was removed (cf Figure 6).

(3) 5 µg of plasmid pTRP501 DNA was cleaved with restriction enzymes ClaI (Boehringer Mannheim, West Germany) and HpaI in 50 µl of a reaction mixture (10 mM Tris-HCl, pH 7.5, 7 mM MgCl$_2$, 100 mM KCl, 7 mM 2-mercaptoethanol, 0.01% bovine serum albumin, 5 units ClaI, 5 units HpaI) at 37°C for 2 hours. Then, 0.5 unit of alkaline phosphatase was added and the reaction was carried out at 65°C for 30 minutes. The reaction mixture was subjected to 0.7% agarose slab gel electrophoresis under the conditions described in Example 3 and a 2.6 kbp DNA fragment was recovered from the gel by the method described in Example 3. Separately, 50 µg of plasmid pTRP501 DNA was cleaved with restriction enzyme HpaI in 200 µl of a reaction mixture (10 mM Tris-HCl, pH 7.5, 7 mM MgCl$_2$, 100 mM KCl, 7 mM 2-mercaptoethanol, 5 units HpaI) at 37°C overnight. Then, 50 units of restriction enzyme TaqI was added and the reaction was continued at 65°C for 2 hours. Using 10% polyacrylamide slab gel, the reaction mixture was electrophoresed in a buffer (89 mM Tris-HCl, 89 mM boric acid, 2.5 mM EDTA, pH 8.3) at 160 V for 1.5 hours. After the electrophoresis, a 32 bp DNA fragment was recovered from the gel by the method described in Example 3.

200 ng of the 2.6 kbp ClaI-HpaI DNA fragment and 1.5 ng of the ClaI-TaqI DNA fragment were mixed and subjected to DNA ligation with T4 DNA ligase under the conditions described in Reference Example (i) (1). Using the reaction mixture, Escherichia coli 294 was transformed, and plasmid pTRP601 DNA was obtained from the ampicillin-resistant transformant (cf Figure 6).

(4) 1 µg of plasmid pTRP601 DNA was cleaved with restriction enzyme PvuII in 20 µl of a reaction mixture (10 mM Tris-HCl, pH 7.5, 7 mM MgCl$_2$, 60 mM NaCl, 7 mM 2-mercaptoethanol, 2 units PvuII) at 37°C for 2 hours. The reaction mixture was deproteinized with phenol and the DNA was precipitated with cold ethanol. Separately, 2 µg of plasmid pBR322 DNA was cleaved with restriction enzymes EcoRI (Takara Shuzo) and AvaI (New England BioLbas) in 20 µl of a reaction mixture (100 mM Tris-HCl, pH 7.5, 7 mM $MgCl_2$, 50 mM NaCl, 7 mM 2-mercaptoethanol, 2 units EcoRI, 2 units AvaI) at 37°C for 2 hours. The reaction mixuture was subjected to 0.7% agarose slab gel electrophoresis under the conditions desdribed in Example 3. After the electrophoresis, a 1.4 kbp EcoRI-AvaI DNA fragment was recovered from the gel by the method described in Example 3.

500 ng of the 1.4 kbp EcoRI-AvaI DNA fragment was filled in with DNA polymerase large fragment (New England BioLabs) in 30 µl of a reaction mixture (40 mM potassium phosphate buffer, pH 7.5, 6.6 mM $MgCl_2$, 1 mM 2-mercaptoethanol, 33 µM dATP, 33 µM dGTP, 33 µM dTTP, 33 µM dCTP, 2 units Klenow's DNA polymerase I) at room temperature for 30 minutes, whereby the cohesive ends were rendered blunt. 200 ng of said 1.4 kbp DNA fragment and 200 ng of PvuII-digested pTRP601 DNA were mixed and subjected to ligation with T4 DNA ligase under the conditions described in Reference Example (i)(1). Escherichia coli 294 was transformed with the reaction mixture, and plasmid pTRP701 DNA was obtained from the ampicillin- and tetracycline-resistant transformant (cf Figure 6).

(5)  2 µg of plasmid pTRP701 DNA was partially digested with diluted restriction enzyme ClaI in 20 µl of a reaction mixture (10 mM Tris-HCl, pH 8.0, 10 mM $MgCl_2$, diluted ClaI) at 37°C for 15 minutes. Following deproteinization with phenol, the DNA was precipitated with ethanol. The cohesive ends of this partially cleaved pTRP 701 DNA was filled in with Klenow's DNA polymerase I under the conditions described in Reference Example (i) (4). Following deproteinization with phenol, the DNA was precipitated with cold ethanol. The DNA was religated with T4 DNA ligase under the conditions described in Reference Example (i) (1). Escherichia coli 294 was transformed with the reaction mixture, and plasmid pTR771 having only one ClaI site as compared with two ClaI sites originally present

in plasmid pTRP701 DNA was obtained from the thus-obtained transformant. The ClaI cleavage site was present immediately after the SD sequence succeeding the trp promoter (cf Figure 6).

(ii)   Construction of rec A promoter-containing expression vector

200 µg of plasmid pMCR611 DNA was cleaved with restriction enzymes BamHI and PstI (Takara Shuzo) in 400 µl of a reaction mixture (10 mM Tris-HCl, pH 8.0, 7 mM MgCl$_2$, 100 mM NaCl, 2 mM 2-mercaptoethanol, 0.01% bovine serum albumin, 20 units BamHI, 20 units PstI) at 37°C overnight, and the reaction mixture was electrophoresed using 1.0% agarose slab gel under the conditions described in Example 3. After the electrophoresis, a 1.25 kbp DNA fragment was recovered from the gel by the method described in Example 3. 30 µg of said 1.25 kpb DNA fragment was cleaved with restriction enzyme HaeIII (Takara Shuzo) in 100 µl of a reaction mixture (10 mM Tris-HCl, pH 7.5, 7 mM MgCl$_2$, 7 mM 2-mercaptoethanol, diluted HaeIII) at 37°C for 15 minutes. The reaction mixture was electrophoresed using 1.2% agarose slab gel under the conditions described in Example 3. Thereafter, 1.06 kbp and 1.19 kbp DNA fragments were respectively recovered from the gel by the method described in Exmaple 3. 2 µg of the 1.06 kbp DNA fragment and 500 ng of 1.19 kbp DNA fragment were respectively filled in with Klenow's DNA polymerase I under the conditions described in Reference Example (i) (4), and, following deproteinization with phenol, the DNA was precipitated with cold ethanol (cf Figure 7).

200 ng of HindIII linker d(CAAGCTTG) (Takara Shuzo) was phosphorylated at the 5'-end with use of T4 polynucleotide kinase (Takara Shuzo) in 50 µl of a reaction mixture (50 mM Tris-HCl, pH 7.6, 10 mM MgCl$_2$, 10 mM 2-mercaptoethanol, 100 µM ATP, 3 units T4 polynucleotide kinase) at 37°C for an hour.

4 ng of the 5'-phosphorylated HindIII linker [5'-P-d(CAAGCTTG)] and 400 ng of the blunt-ended 1.06 kbp DNA fragment were mixed and subjected to ligation with T4 DNA ligase under the conditions described in Reference Example (i) (1). To the reaction mixture, there was then added 20 µl of a buffer (10 mM Tris-HCl, pH 8.0, 7 mM MgCl$_2$, 100 mM NaCl, 2 mM 2-mercaptoethanol, 0.01% bovine serum albumin) containing 10 units each of restriction enzymes BamHI and HindIII, and the reaction was allowed to proceed at 37°C overnight, whereby both the ends were converted to cohesive ends.

2 ng of the above 5'-phosphorylated HindIII linker and 200 ng of the blunt-ended 1.19 kbp DNA were mixed and subjected to ligation under the same conditions as mentioned above, whereby both the ends of the DNA were converted to cohesive ends.

Separately, 2 µg of plasmid pBR322 DNA was cleaved with restriction enzymes BamHI and HindIII in 20 µl of a reaction mixture (10 mM Tris-HCl, pH 8.0, 7 mM MgCl$_2$, 100 mM NaCl, 2 mM 2-mercaptoethanol, 0.01% bovine serum albumin, 3 units BamHI, 3 units HindIII) at 37°C for 2 hours, and, following addition of 0.2 unit of alkaline phosphatase, the reaction was continued at 65°C for 30 minutes. The reaction mixture was electrophoresed using 1.0% agarose slab gel under the conditions described in Example 3, and a 4.0 kbp DNA fragment was recovered from the gel by the method of Exmaple 3.

Then, 500 ng of the 4.0 kbp DNA and 200 ng of the blunt-ended 1.06 kbp DNA were mixed and subjected to ligation with T4 DNA ligase under the conditions described in Reference Example (i) (1). Escherichia coli 294 was transformed with the reaction mixture, and a rec A promoter-containing plasmid pREC101 was obtained from the ampicillin-resistant transformant. Furthermore, 500 ng of the 4.0 kbp DNA and 100 ng of the blunt-ended 1.19 kbp DNA were mixed and subjected to the same reaction as above to give a rec A promoter-containing plasmid pREC201.

### Example 4

Construction of recombinant DNA molecules for HBsAg/adw gene expression and transformation of Escherichia coli with the recombinant DNA molecules

(i)    Construction of a fused gene of HBsAg gene with
       trp E gene

(1)    1 µg of plasmid pTRP202 DNA was cleaved with
restriction enzyme BglII in 20 µl of a reaction mixture
(10 mM Tris-HCl, pH 7.5, 7 mM $MgCl_2$, 60 mM NaCl, 7 mM 2-
mercaptoethanol, 2 units BglII) at 37°C for 2 hours.  Then,
0.1 unit of alkaline phosphatase was added and the reaction
was continued at 65°C for 30 minutes.  Thereafter, following
deproteinization with phenol, the DNA was precipitated with
cold ethanol (BglII-digested pTRP202).

500 ng of the BglII-digested pTRP202 and 500 ng of the
HBsAg gene fraction B were mixed and subjected to ligation
with T4 DNA ligase under the conditions described in Reference
Example (i) (1).  Escherichia coli 294 was transformed with
the reaction mixture, and the plasmid DNA was extracted from
the ampicillin-resistant transformant by the method of
Birnboim and Doly and analyzed for restriction enzyme cleavage
pattern.  As a result, a transformant (294/pTRP ES-3) carrying plasmid pTRP ES-3 with fragment B inserted therein in
the same direction as the gene (trp E) coding for anthranilic
acid synthetase component I in the tryptophan operon was
isolated.

(2)    2 µg of HBsAg gene fragment E was filled in with
Klenow's DNA polymerase I under the conditions described
in Reference Example (i) (4), whereby the cohesive ends were
rendered blunt.  1 µg of blunt-ended fragment E was ligated
with 50 ng of BglII linker phosphorylated at the 5'-end by
the method described in Reference Exmaple (ii) [5'-P-d
(CAGATCTG)] (New England BioLabs), with T4 DNA ligase under
the conditions described in Reference Example (i) (1).  To
the reaction mixture was added an excess of restriction
enzyme BglII so as to cause cohesive-end formation and
simultaneous decomposition of the remaining BglII linker.
Then, following deproteinization with phenol, the DNA was
precipitated with cold ethaol.

Then, 1 µg of fragment E with said linker ligated thereto was ligated to 2 µg of BalII-digested pTRP202 DNA with T4 DNA ligase under the conditions described in Reference Example (i) (1). Escherichia coli 294 was transformed using the reaction mixture, and a strain (294/pTRP ES-11) carrying plasmid pTRP ES-11 with fragment E inserted therein in the same direction as the gene (trp E) coding for anthranilic acid synthetase component I in the tryptophan operon was isolated as a result of transformant examination performed by the method described in Example 4 (i) (1).

(3)    2 µg of HBsAg gene fragment F and 2 µg of BglII-digested pTRP202 DNA were respectively filled in with Klenow's DNA polymerase I under the conditions described in Reference Example (i) (4), whereby the cohesive ends were rendered blunt.  Both the DNAs were then mixed and subjected to ligation with T4 DNA ligase under the conditions described in Reference Example (i) (1).  Using the reaction mixture, Escherichia coli 294 was transformed, and a strain (294/pTRP ES-21) carrying plasmid pTRP ES-21 with fragment F inserted therein in the same direction as the gene (trp E) coding for anthranilic acid synthetase component I in the tryptophan operon was isolated as a result of transformant examination conducted by the method described in Example 4 (i) (1).

(ii)    Construction of a fused gene of HBsAg gene with trp D gene

(1)    1 µg of plasmid pTRP501 DNA was cleaved with restriction enzyme HindIII in 20 µl of a reaction mixture (10 mM Tris-HCl, pH 7.5, 7 mM $MgCl_2$, 60 mM NaCl, 2 units HindIII) at 37°C for 2 hours.  Then, 0.1 unit of alkaline phosphatase was added and the reaction was continued at 65°C for 30 minutes.  After the reaction, deproteinization with phenol was performed and the DNA was precipitated by adding cold ethanol (HindIII-digested pTRP501).

2 µg of HBsAg gene fragment A was filled in with Klenow's DNA polymerase I under the conditions described in Reference Example (i) (4) so as to render the cohesive ends blunt.  1 µg of blunt-ended fragment A and 50 ng of 5'-phosphorylated HindIII linker [5'-P-d(CAAGCTTG)] prepared by the method described in Reference Example (ii) were mixed and subjected to DNA ligation with T4 DNA ligase under the conditions described in Reference Example (i) (1). To the reaction mixture was added an excess of restriction enzyme HindIII for cohesive end formation and decomposition of the remaining HindIII linker. Following deproteinization with phenol, the DNA was precipitated with cold ethanol.

Then, 1 µg of fragment A with said linker joined thereto and 1 µg of HindIII-digested pTRP501 DNA were ligated to each other with T4 DNA ligase under the conditions described in Reference Example (i) (1). Escherichia coli 294 was transformed using the reaction mixture, and a strain (294/ pTRP DS-1) carrying plasmid pTRP DS-1 with fragment A inserted therein in the same direction as the gene (trp D) coding for anthranilic acid synthetase component II in the tryptophan operon was isolated as a result of transformant examination carried out by the method described in Example 4 (i) (1).

(2)    1 µg of HBsAg gene fragment E was ligated to 50 ng of 5'-phosphorylated HindIII linker [5'-P-d(CAAGCTTG)] prepared by the method described in Reference Example (ii), with T4 DNA ligase under the conditions described in Reference Example (i) (1). To the reaction mixture, an excess of restriction enzyme HindIII was added for cohesive end formation and decomposition of the remaining HindIII linker. Then, following deproteinization with phenol, the DNA was precipitated with cold ethanol.

Then, 200 ng of fragment E with said linker joined thereto and 200 ng of HindIII-digested pTRP501 DNA were subjected to ligation with T4 DNA ligase under the conditions described in Reference Example (i) (1). Using the reaction mixture, Escherichia coli 294 was transformed, and a strain (294/pTRP DS-33) carrying plasmid pTRP DS-33 with fragment E inserted therein in the same direction as the gene (trp D) coding for anthranilic acid synthetase component II in the tryptophan operon was isolated as a result of transformant examination performed by the method described in Example 4 (i) (1).

(3)    2 µg of HBsAg fragment F was filled in with Klenow's DNA polymerase I under the conditions described in Reference Example (i) (4) so as to render the fragment blunt-ended.  1 µg of said blunt-ended fragment F and 50 ng of 5'-phosphorylated HindIII linker [5'-P-d(CAAGCTTG)] were subjected to ligation with T4 DNA ligase under the conditions described in Reference Example (i) (1).  An excess of restriction enzyme HindIII was added to the reaction mixture for cohesive end formation and decomposition of the remaining HindIII linker.  Then, following deproteinization with phenol, the DNA was precipitated with cold ethanol.

Then, 200 ng of fragment F with said linker joined thereto and 200 ng of HindIII-digested pTRP501 DNA were subjected to ligation with T4 DNA ligase under the conditions described in Reference Example (i) (1).  Using the reaction mixture, Escherichia coli 294 was transformed, and a strain (294/pTRP DS-42) carrying plasmid pTRP DS-42 with fragment F inserted therein in the same direction as the gene (trp D) coding for anthranilic acid synthetase component II in the tryptophan operon was isolated as a result of transformant examination performed by the method described in Example 4 (i) (1).

(iii)  Construction of a fused gene of HBsAg gene with β-
       lactamase gene (bla)

(1)    1 µg of plasmid pTRP701 DNA was cleaved with restriction enzyme PstI in 20 µl of a reaction mixture [20 mM Tris-HCl, pH 7.5, 1u mM $MgCl_2$, 50 mM $(NH_4)_2SO_4$, 0.01% bovine serum albumin, 2 units PstI] at 37°C for 2 hours.  Then, following addition of 0.1 unit of alkaline phosphatase, the reaction was continued at 65°C for 30 minutes.  After the reaction, deproteinization was performed with phenol and the DNA was precipitated by adding cold ethanol (PstI-

·digested pTRP701).

2 µg of HBsAg gene fragment A was filled in .with Klenow's DNA polymerase I under the conditions described in Reference Example 3 (i) (4) so as to render the cohesive ends blunt. 1 µg of blunt-ended framgent A and 50 ng of PstI linker (New England BioLabs) phosphorylated. at the 5'-end by the method described in Reference Example (ii) [5'-P-d(GCGTCAGC)] were ligated together with T4 DNA ligase under the conditions described in Reference Example (i) (1). An excess of restriction enzyme PstI was added to the reaction mixture for cohesive end formation and decomposition of the remaining PstI linker. Thereafter, following deproteinization with phenol, the DNA was precipitated with cold ethanol.

Then, 200 ng of fragment A with said linker joined thereto and 200 ng of PstI-digested pTRP701 were ligated together with T4 DNA ligase under the conditions described in Reference Example (i) (1). Using the reaction mixture, Escherichia coli 294 was transformed, and a strain (294/pTRP LS-2) carrying plasmid pTRP LS-2 with fragment A inserted therein in the same direction as the gene (bla) coding for β-lactamase in the ampicillin registance gene operon was isolated as a result of transformant examination by the method described in Example 4 (i) (1).

(2) 1 µg of HBsAg gene fraction E and 50 ng of 5'-phosphorylated PstI linker [5'-P-d(GCTGCAGC)] prepared by the method described in Reference Example (ii) were ligated together with T4 DNA ligase under the conditions described in Reference Example (i) (1). An excess of restriction enzyme PstI was added to the reaction mixture for cohesive end formation and decomposition of the remaining PstI linker. Thereafter, following deproteinization with phenol, the DNA was precipitated with cold ethanol.

Then, 200 ng of fragment E with PstI linker joined thereto and 200 ng of PstI-digested pTRP701 were ligated together with T4 DNA ligase under the conditions described in Reference Example (i) (1). Using the reaction mixture, Escherichia coli 294 was transformed, and a strain (294/pTRP LS-31) carrying plasmid pTRP LS-31 with fragment E inserted therein in the same direction as the gene (bla) coding for β-lactamase in the ampicillin resistance gene operon was isolated as a result of transformant examination by the method described in Example 4 (i) (1).

(iv)　Construction of a fuged gene of HBsAg gene with with rec A protein gene

(1)　1 µg of plasmid pREC101 DNA was cleaved with restriction enzyme HindIII in 20 µl of a reaction mixture (10 mM Tris-HCl, pH 7.5, 7 mM MgCl$_2$, 60 mM ·NaCl, 2 units HindIII) at 37°C for 2 hours. Then, 0.1 unit of alkaline phosphatase was added and the reaction was contineud at 65°C for 30 minutes. Thereafter, following deproteinization with phenol, the DNA was precipitated by adding cold ethanol (HindIII-digested pREC101).

2 µg of HBsAg fragment A was filled in with Klenow's DNA polymerase I under the conditions described in Reference Example (i) (4) to render the cohesive ends blunt. 1 µg of blunt-ended fragment A and 50 ng of HindIII linker (RRL) phosphorylated at the 5'-end by the method described in Reference Example (ii) [5'P-d(GCAAGCTTGC)] were ligated together with　T4 DNA ligase under the conditions described in Reference Example (i) (1). To the reaction mixture, an excess of restriction enzyme HindIII was added for cohesive end formation and decomposition of the remaining HindIII linker. Thereafter, following deproteinization with phenol, the DNA was precipitated with cold ethanol.

·Then, 200 ng of fragment A with said linker joined thereto and 200 ng of HindIII-digested pREC101 DNA were ligated together with T4 DNA ligase under the conditions described in Reference Example (i) (1). Using the reaction mixture, Escherichia coli 294 was transformed, and a strain (294/pREC RS-3) carrying plasmid pREC RS-3 with fragment A inserted therein in the same direction as the gene coding for rec A protein in the rec A operon was isolated as a result of trnasformant examination by the method described in Example 4 (i) (1).

(2)     1 μg of HBsAg gene fragment E and 50 ng of 5'-phosphorylated HindIII linker [5'-P-d(GCAAGCTTGC)] prepared by the method described in Reference Example (ii) were ligated together with T4 DNA ligase under the conditions described in Reference Example (i) (1). To the reaction mixture, an excess of restriction enzyme HindIII was added for cohesive end formation and decomposition of the remaining HindIII linker. Thereafter, following deproteinization with phenol, the DNA was precipitated with cold ethanol.

Then, 200 ng of fragment E with said linker joined thereto and 200 ng of HindIII-digested pREC101 DNA were ligated together with T4 DNA ligase under the conditions described in Reference Example (i) (1). Using the reaction mixture, Escherichia coli 294 was transformed, and a strain (294/pREC RS-11) carrying plasmid pREC RS-11 with fragment E inserted therein in the same direction as the gene coding for rec A protein in the rec A operon was isolated as a result of transformant examination performed by the method described in Example 4 (i) (1).

(v)     Construction of plasmid for direct expression of HBsAg gene and fragment thereof

(1)     1 μg of plasmid pTRP771 DNA was cleaved with restriction enzyme ClaI in 20 μl of a reaction mixture (10 mM Tris-HCl, pH 8.0, 10 mM MgCl$_2$, 2 units ClaI) at 37°C for 2 hours. Then, 0.1 unit of alkaline phosphoatase was added and the reaction was continued at 65°C for 30 minutes. Thereafter, following deproteinization with phenol, the DNA was precipitated by adding cold ethanol (ClaI-digested pTRP771).

2 μg of HBsAg gene fragment A was filled in with Klenow's DNA polymerase I under the conditions described in Reference Example (i) (4) to render the cohesive ends blunt. 1 μg of blunt-ended fragment A and 50 ng of BamHI linker (Takara Shuzo) phosphorylated at the 5'-end by the method described in Reference Example (ii) [5'-P-d(CCGGATCCGG)] were ligated together with T4 DNA ligase under the conditions described in Reference Example (i) (1). To the reaction mixture, an excess of restriction enzyme HpaII (New England BioLabs) was added for cohesive end formation and decomposition of the remaining BamHI linker. Thereafter, following deproteinization with phenol, the DNA was precipitated with cold ethanol.

Then, 200 ng of fragment A with said linker joined thereto and 200 ng of ClaI-digested pTRP771 DNA were ligated together with T4 DNA ligase under the conditions described in Reference Example (i) (1). Using the reaction mixture, Escherichia coli 294 was transformed, and a strain (294/pTRP S-5) carrying plasmid pTRP S-5 with fragment A inserted therein in the same direction as the trp promoter.

(2) 1 µg of HBsAg gene fragment E and 50 ng of synthetic adapter phosphorylated at the 5'-end by the method described in Reference Example (ii)

5'pCCGCATCGAT3'

CGTAGCTAp

were ligated together with T4 DNA ligase under the conditions described in Reference Example (i) (1). After the remaining adapter was decomposed by adding an excess of restriction enzyme ClaI to the reaction mixture, deproteinization was performed with phenol and the DNA was precipitated with cold ethanol. The above adapter was chemically synthesized by the phosphotriester method [R. Crea et al., Proc. Natl. Acad. Sci. USA, 75, 5765-5769 (1978) which is hereby incorporated by reference].

Then, 200 ng of fragment E with said adapter joined thereto and 200 ng of ClaI-digested pTRP771 DNA were ligated together with T4 DNA ligase under the conditions described in Reference Example (i) (1). Using the reaction mixture, Escherichia coli 294 was transformed, and a strain (294/pTRP S-12) carrying plasmid pTRP S-12 with fragment E inserted therein in the same direction as the trp promoter

- 33 -

was isolated as a result of transformant examination by the method described in Example 4 (i) (1).

## Example 5

### Expression of HBsAg/adw gene in Escherichia coli

Each of the transformants containing HBsAg gene expression plasmids obtained in Example 4 was grown in M-9 medium containing 0.5% glucose and 0.5% Casamino Acids at 37°C for 6 hours. The cells were collected and washed with a buffer (30 mM Tris-HCl, pH 8.0, 50 mM NaCl, 5 mM EDTA). The cells were suspended in a lysing solution containing 10 mM Tris-HCl, pH 8.0, 5 mM EDTA, 1 mM phenylmethylsulfonyl fluoride and 0.5 mg/ml lysozyme and lysed by repeating freezing and thawing three times. The lysate was centrifuged at 4°C and 15,000 rpm for 30 minutes. The HBsAg activity of the supernatant was assayed using an Auslia II-125 kit. The results obtaining in this manner are shown below in Table 2. The yield of peptide showing HBsAg antigenicity was calculated approximately at 2,000-4,000 HBsAg molecules per $10^3$ cells.

### Table 2

| Transformant | Number of HBsAg molecules per $10^3$ cells |
|---|---|
| E.coli 294/pTRP ES-3 | 2200 |
| E.coli 294/pTRP ES-11 | 2600 |
| E.coli 294/pTRP ES-21 | 2500 |
| E.coli 294/pTRP DS-1 | 3800 |
| E.coli 294/pTRP DS-33 | 4000 |
| E.coli 294/pTRP DS-42 | 3900 |
| E.coli 294/pTRP LS-2 | 2700 |
| E.coli 294/pTRP LS-31 | 3500 |
| E.coli 294/pREC RS-3 | 2200 |
| E.coli 294/pREC RS-11 | 2700 |
| E.coli 294/pTRP S-5 | 2500 |
| E.coli 294/pTRP S-12 | 3000 |

To the supernatant of the lysate of Escherichia coli

294/pTRP DS-33 obtained above was added thereto ammonium sulfate to 80% saturation. The resulting precipitates were collected by centrifugatoin at 5°C, dialyzed against saline and lyophilized to obtain HBsAg/adw as a crude powder.

E.Coli W 3110 and RR$_1$ are known strains referred to respectively in Bacteriol. Rev. Vol 36, 525-557 (1972) and methods in Enzymology Vol 68, 262 (1979) Academic Press. E.Coli W3110 is available from the Institute for Fermentation, Osaka, Japan under deposit number IFO 12713. E.Coli RR$_1$ is available from the American Type Culture Collection 12301 Parklawn Drive, Rockville Md 20852 USA under 'the deposit' number ATCC No. 31343.

What is claimed is:

1.  A DNA which contains a structural gene coding for
hepatitis B virus subtype adw surface antigen.

2.  A DNA according to Claim 1, wherein the structural
gene coding for the surface antigen has the base sequence
shown in Figure 4 as covering bases 127-807 or a fragment
thereof.

3.  A DNA according to Claim 1 or 2, wherein the structural
gene coding for the surface antigen codes for the polypeptide
shown in Figure 4 or a polypeptide equivalent thereto in
immunological or biological activity.

4.  A DNA according to any one of Claims 1-3, which is in
the form of a recombinant DNA consisting of a vector DNA
and the structural gene coding for the surface antigen inserted
in said vector DNA.

5.  A DNA according to any one of Claims 1-4, which contains
the gene coding for the surface  antigen as inserted in the
gene coding for anthranilic acid synthetase component I or
anthranilic acid synthetase component II in the tryptophan
operon, the gene coding for rec A protein in the rec A operon
or the gene coding for β-lactamase in the ampicillin resistance gene operon, or downstream from the tryptophan promoter
or rec A promoter.

6.  A DNA according to any one of Claims 1-5, wherein the
base sequence shown in Figure 4 as covering bases 127-807
or a fragment thereof with ATG joined to the 5'-end without
any reading frame shift is inserted downstream from the
tryptophan promoter or rec A promoter.

7.  A method of producing a recombinant DNA containing a

- 36 -

structural gene coding for hepatitis B virus subtype adw
surface antigen, which comprises subjecting a vector DNA and a
hepatitis B virus subtype adw DNA from a Dane particle of
hepatitis B virus  subtype adw, after rendering the single-
stranded portion of the latter DNA double-stranded by
endogenous DNA synthesis reaction, each to restriction
enzyme cleavage, mixing the cleavage products and thereby
causing insertion of the hepatitis B virus subtype adw DNA
or a fragment thereof at the cleavage site in said vector
DNA.

8.    A method according to Claim 7, wherein the structural
gene coding for the surface antigen has the base sequence
shown in Figure 4 as covering bases 127-807 or a fragment
thereof.

9.    A method according to Claim 7 or 8, wherein the structural gene coding for the surface antigen codes for the
polypeptide shown in Figure 4 or a polypeptide equivalent
thereto in immunological or biological activity.

10.    A method according to any one of Claims 7-9, wherein
the gene coding for the surface antigen is inserted in the
gene coding for anthranilic acid synthetase component I or
anthranilic acid synthetase component II in the tryptophan
operon, the gene coding for rec A protein in the rec A
operon or the gene  coding for β-lactamase in the ampicillin
resistance gene operon, or downstream from the tryptophan
promoter or rec A promoter.

11.    A method according to any one of Claims 7-10 wherein
the base sequence shown in Figure 4 as covering bases 127-
807 or a fragment thereof with ATG joined to the 5'-end
without any reading frame shift is inserted downstream from
the tryptophan promoter or rec A promoter.

12. A transformant which contains a recombinant DNA containing a structural gene coding for hepatitis B virus subtype
adw surface antigen.

13. A transformant according to Claim 12, wherein the
structural gene coding for the surface antigen has the base
sequence shown in Figure 4 as covering bases 127-807 or a
fragment thereof.

14. A transformant according to Claim 12 or 13, wherein
the structural gene coding for the surface antigen codes
for the polypeptide shown in Figure 4 or a polypeptide
equivalent thereto in immunological or biological activity.

15. A transformant according to any one of Claims 12-14
which contains a recombinant DNA containing the structural
gene coding for the surface antigen as inserted in the gene
coding for anthranilic acid synthetase component I or
anthranilic acid synthetase component II in the tryptophan
operon, the gene coding for rec A protein in the rec A
operon or the gene coding for β-lactamase in the ampicillin
resistance gene operon, or downstream from the tryptophan
promoter or rec A promoter.

16. A transformant according to any one of Claims 12-15,
wherein the base sequence shown in Figure 4 as covering
bases 127-807 or a fragment thereof with ATG joined to the
5'-end without any reading frame shift is inserted downstream
from the tryptophan promoter or rec A promoter.

17. A transformant according to any one of Claims 12-16, which
is Escherichia coli.

18. A method of producing hepatitis B virus subtype adw
surface antigen or a polypeptide equivalent thereto in
immunological or biological activity which comprises growing

- 38 -

a transformant with a recombinant DNA containing a structural gene coding for hepatitis B virus subtype adw surface antigen in an expression system until hepatitis B virus subtype adw surface antigen or a polypeptide equivalent thereto in immunological or biological activity is produced and accumulated in the culture broth, and recovering the accumulated surface antigen or the polypeptide from the culture broth.

19.    A method according to Claim 18, wherein the structural gene coding for the surface antigen has the base sequence shown in Figure 4 as covering bases 127-807 or a fragment thereof.

20.    A method according to Claim 18 or 19, wherein the structural gene coding for the surface antigen codes for the polypeptide shown in Figure 4 or a polypeptide equivalent thereto in immunological or biological activity.

21.    A method according to any one of Claims 18-20, wherein the transformant contains a recombinant DNA containing the structural gene coding for the surface antigen as inserted in the gene coding for anthranilic acid synthetase component I or anthranilic acid synthetase component II in the tryptophan operon, the gene coding for rec A protein in the rec A operon or the gene coding for β-lactamase in the ampicillin resistance gene operon or downstream from the tryptophan promoter or rec A promoter.

22.    A method according to any one of Claims 18-21, wherein the base sequence shown in Figure 4 as covering bases 127-807 or a fragment thereof with ATG joined to the 5'-end without any reading frame shift is inserted downstream from the tryptophan promoter or rec A promoter.

23.    A method according to any one of Claims 18-22, wherein the transformant is Escherichia coli.

24.    A polypeptide of the amino acid sequence shown in
Figure 4.

25.    Hepatitis B virus subtype adw surface antigen or a
polypeptide equivalent thereto in immunological or biological
activity, which is obtainable by a method claimed in any one
of Claims 18-23.

26.    A composition of matter which comprises hepatitis B
virus subtype adw surface antigen or a polypeptide equivalent
thereto in immunological or biological activity, the balance
of said composition comprising soluble microbial protein.

Fig.1

0068719

07·07·82

Escherichia coli + 1776 / pBR 322 - EcoRI / HBV 933

pBR322 DNA

Plasmid

pBR 322

HBV 1.4 kbp DNA

Bam HI digestion

Bam HI digestion

HBV 1.4 kbp DNA

HBs-Ag gene

5'GATCC ——————— G 3'
  3'G ═══════════ CCTAG5'

G 3'
CCTAG5'

5'GATCC
3'G

annealing
T4 DNA ligase

# Fig.2

pBR322

G GATCC
C CTAG G

G GATCC
CCTAG G

HBV DNA

Escherichia coli + 1776

Amp.r (TetS)

Escherichia coli + 1776 / pBR322 - Bam HI / HBV 971

0068719

0.03kb Bam HI    1.4kb Bam HI
Eco RI

1.8kb

Amp$^r$

4.0kb    Tet$^r$    Eco RI
0.4kb
Bam HI

pBR322 – Eco RI / HBV 933

## Fig.3

base    500    1000    1355

HBs Ag gene

Bam HI

Sau 3A

Xba I

Hpa I

Hinc II

Fragment A

Fragment B

Fragment E    ## Fig.5

Fragment F

5' 1
GATCCCAGAGTCAGGGGGTCTGTATCTTCCTGCTGGTGGCTCCAGTTCAGGAACAGTAAACCCTGCTCCGAATATTGCCTCTCACATCTCGTCAATCTCCGCGAGGACTG

127
GGGACCCTGTGACGATC ATG GAG AAC ATC ACA TCA GGA TTC CTA GGA CCC CTG CTC GTG TTA CAG GCG GGG TTT TTC TTG TTG ACA
Met Glu Asn Ile Thr Ser Gly Phe Leu Gly Pro Leu Leu Val Leu Gln Ala Gly Phe Phe Leu Leu Thr

200
AGA ATC CTC ACA ATA CCG CAG AGT CTA GAC TCG TGG TGG ACT TCT CTC AAT TTT CTA GGG GGA TCA CCC GTG TGT CTT GGC CAA
Arg Ile Leu Thr Ile Pro Gln Ser Leu Asp Ser Trp Trp Thr Ser Leu Asn Phe Leu Gly Gly Ser Pro Val Cys Leu Gly Gln

300
AAT TCG CAG TCC CCA ACC TCC AAT CAC TCA CCA ACC TCC TGT CCT CCA ATT TGT CCT GGT TAT CGC TGG ATG TGT CTG CGG CGT
Asn Ser Gln Ser Pro Thr Ser Asn His Ser Pro Thr Ser Cys Pro Pro Ile Cys Pro Gly Tyr Arg Trp Met Cys Leu Arg Arg

400
TTT ATC ATA TTC CTC TTC ATC CTG CTG CTA TGC CTC ATC TTC TTA TTG GTT CTT CTG GAT TAT CAA GGT ATG TTG CCC GTT TGT
Phe Ile Ile Phe Leu Phe Ile Leu Leu Leu Cys Leu Ile Phe Leu Leu Val Leu Leu Asp Tyr Gln Gly Met Leu Pro Val Cys

500
CCT CTA ATT CCA GGA TCA ACA ACA ACC AGT ACG GGA CCA TGC AAA ACC TGC ACG ACT CCT GCT CAA GGC AAC TCT AAG TTT CCC
Pro Leu Ile Pro Gly Ser Thr Thr Thr Ser Thr Gly Pro Cys Lys Thr Cys Thr Thr Pro Ala Gln Gly Asn Ser Lys Phe Pro

600
TCA TGT TGC TGT ACA AAA CCT ACG GAT GGA AAT TGC ACC TGT ATT CCC ATC CCA TCG TCC TGG GCT TTC GCA AAA TAC CTA TGG
Ser Cys Cys Cys Thr Lys Pro Thr Asp Gly Asn Cys Thr Cys Ile Pro Ile Pro Ser Ser Trp Ala Phe Ala Lys Tyr Leu Trp

GAG TGG GCC TCA GTC CGT TTC TCT TGG CTC AGT TTA CTA GTG CCA TTT GTT CAG TGG TTC GTA GGG CTT TCC CCC ACT GTT TGG
Glu Trp Ala Ser Val Arg Phe Ser Trp Leu Ser Leu Leu Val Pro Phe Val Gln Trp Phe Val Gly Leu Ser Pro Thr Val Trp

700
CTT TCA GCT ATA TGG ATG ATG TGG TAT TGG GGG CCA AGT CTG TAC AGC ATC GTG AGT CCC TTT ATA CCG CTG TTA CCA ATT TTC
Leu Ser Ala Ile Trp Met Met Trp Tyr Trp Gly Pro Ser Leu Tyr Ser Ile Val Ser Pro Phe Ile Pro Leu Leu Pro Ile Phe

800     807
TTT TGT CTC TGG GTA TAC ATT TAA ACCCTAACAAAACAAAAAGATGGGGGTTATTCCCTAAACTTCATGGGCTACATAATTGGAAGTTGGGGAACTTTGCCACA
Phe Cys Leu Trp Val Tyr Ile oc

900
GGATCAAACACTGTTTTAGAAAACTTCCTGTTAACAGGCCTATTGATTGGAAAGTATGTCAAAGAATTGTGGGTCTTTTGGGCTTTGCTGCTCCATTTACACAATGTGGAT

1000                                                                                                                      1100
ATCCTGCCTTAATGCCTTTGTATGCATGTATACAAGCTAAACAGGCTTTCACTTTCTCGCCAACTTACAAGGCCTTTCTAAGTAAACAGTACATGAACCTTTACCCCGTTG

1200
CTCGGCAACGGCCTGGTCTGTGCCAAGTGTTTGCTGACGCAACCCCCACTGGCTGGGGCTTAGCCATAGGCCATCAGCGCATGCGTGGAACCTTTGTGGCTCCTCTGCCGA

1300
TCCATACTGCGGAACTCCTAGCCGCTTGTTTTGCTCGCAGCCGGTCTGGAGCAAAGCTCATCGGAACTGACAATTCTGTCGTCCTCTCGCGGAAATATACATCATTTCCAT

1355
GGCTGCTAGGCTGTACTGCCAACTG 3'

Fig.4

Fig.6

Fig. 7